Europäisches Patentamt

European Patent Office

Office européen des brevets

(11) Publication number: **0 387 027**
**A2**

## (12) EUROPEAN PATENT APPLICATION

(21) Application number: **90302421.4**

(22) Date of filing: **07.03.90**

(51) Int. Cl.5: **G01N 33/68, C12P 21/08, C07K 15/06, G01N 33/543**

The microorganism(s) has (have) been deposited with American Type Culture Collection under number(s) HB-9765.

(30) Priority: **07.03.89 US 320023**
**20.02.90 US 482365**

(43) Date of publication of application:
**12.09.90 Bulletin 90/37**

(84) Designated Contracting States:
**AT BE CH DE DK ES FR GB GR IT LI LU NL SE**

(71) Applicant: **ADEZA BIOMEDICAL CORPORATION**
**1240 Elko Street**
**Sunnyvale, California 94089(US)**

(72) Inventor: **Teng, Nelson N. H.**
**24 Bluebell Lane**
**Hillsborough, California 94010(US)**
Inventor: **Gunupudi, Subba Rao**
**660 Moreno Lane**
**Santa Clara, California 95050(US)**
Inventor: **Feingersh, Diane**
**204 Appian Way**
**Union City, California 94587(US)**

(74) Representative: **Stuart, Ian Alexander et al**
**MEWBURN ELLIS 2 Cursitor Street**
**London EC4A 1BQ(GB)**

(54) **Endometriosis diagnosis methods and reagents.**

(57) A method for determining the presence of endometriosis in a patient comprises contacting a sample from the patient with an anti-(endometriosis) antibody, antigen, or endometriosis antiidiotype antibody, and determining binding of antibody or antigen with a component of the sample. The patient sample can be a fluid sample such as a blood serum sample, or it can be a tissue sample or cell smear. Reagents and immunoassay kits for determining endometriosis are described.

EP 0 387 027 A2

## ENDOMETRIOSIS DIAGNOSIS METHODS AND REAGENTS

This invention relates to the in vitro diagnosis of endometriosis, and to methods and reagents therefor.

Endometriosis is a disease state in which tissues normally found lining the uterus proliferate in other areas of the body, such as within the abdominal cavity. These proliferated tissues respond to monthly endocrine cycles, resulting in abdominal bleeding, adhesions, dysmenorrhea, and infertility.

Preliminary diagnosis of endometriosis is generally made based upon a patient's history of infertility, dysmenorrhea and unexplained pelvic pain. Confirmation of the preliminary diagnosis is made using laproscopy, an invasive procedure in which an incision is made into the abdominal cavity and tissues are visualized and biopsied. There is no currently available simple, noninvasive in vitro diagnostic method, and no method by which endometriosis is detected prior to onset of symptomology.

Endometriosis appears to have some characteristics of autoimmune disease. Antibodies to normal endometrial tissues have been found in the serum of patients with endometriosis, as reported by Mathur et al, *Clin.Exp.Immunol.* **50**:259 (1982); Badawy et al, *Obstet.Gynecol.* **63**:271 (1984); Wild et al, *Am.J.Reprod.Immunol.Microbiol.* **8**:84 (1985), and *Am.J.Reprod.Immunol.Microbiol.* **13**:62 (1987); Chihal et al, *Fertil.Steril.* **46**:408 (1986); and Meek et al, *Am.J.Obstet.Gynecol.* **158**:1365 (1988). A number of antigens including phospholipids, nucleic acids and histones were tested for detecting autoantibodies, and no useful clinical correlations could be found. Mathur et al, *Fertil. Steril.* **50**:860 (1988) reported that two antigens with molecular weights of 34 KD and 26 KD might be associated with endometriosis. Wild et al (supra) reported the use of fixed endometrial, ovarian and breast carcinoma cell lines as antigen sources and reported good clinical correlations. The antigen was speculated to be an epithelial cellular antigen, and more specifically, a cytoplasmic component of the cell. However, no specific epithelial antigen was identified.

Binding of serum antibodies to an immortalized endometrial carcinoma cell line has been reported by Wild et al, *Eighth Ann.Symp.Am.Soc.Immuno.Reprod.*, Portland, Maine, June 15-19 (1988). No endometriosis-specific antigen or anti-(endometriosis) antibody has been reported.

An ovarian carcinoma marker, CA 125, which is a high molecular weight glycoprotein present in endocervix, endometrium, fallopian tube, pleura and pericardium was found to be present in elevated levels in sera of patients with advanced endometriosis as reported by Barbieri et al, *Fertil. Steril.* **45**:630 (1986). However, serum levels of CA 125 increase in all patients during menstruation and has not been found to be a reliable marker to detect endometriosis.

Telimaa et al, *Am. J. Obstet. Gynecol.* **161**:866 (1989) reported elvated levels of endometrial secretory protein PP14 in patients with advanced endometriosis. In mild endometriosis, the levels of this protein did not increase significantly from those found in apparently healthy control subjects.

L.H. Smith et al, *J.Immunol.Methods.* **105**:263-273 (1987) reported generation of hybridomas producing human monoclonal antibodies (hMAbs) to ovarian cancer-associated antigens using patient lymphocytes and the heteromyeloma fusion partner SHM-D33 (U.S. Patent 4,574,116). Among the hMAbs produced was MS2B6 hMAb. The publication does not report any binding with endometriosis tissue.

The method of this invention for determining the presence of endometriosis in a patient comprises contacting a sample from the patient with an anti-(endometriosis) antibody, and determining binding of the antibody with a component of the sample. The sample can be a body fluid, especially a blood serum sample, or a tissue sample, such as a cell smear or biopsy. The anti-(endometriosis) antibody can be labeled. The anti-(endometriosis) antibody can be polyclonal or monoclonal. The anti-(endometriosis) antibody can be a human monoclonal antibody. A preferred monoclonal antibody is human MS2B6 IgM anti-(endometriosis) antibody [produced by ATCC (American Type Culture Collection, 12301 Parklawn Drive, Rockville, Maryland 20852, USA) HB 9765].

The present invention also provides a method for determining the presence of endometriosis which detects anti-(endometriosis) antibodies in a patient, and comprises contacting a blood serum or blood plasma sample from the patient with endometriosis antigen or endometriosis antiidiotype antibody, and determining binding of endometriosis antigen or endometriosis antiidiotype antibody with antibody in the sample. The endometriosis antigen or endometriosis antiidiotype antibody may be bound to a support. The endometriosis antigen or endometriosis antiidiotype antibody may be labeled. Preferred endometriosis antigens include EA 101A, EA 101B, and EA 101C antigen.

In the biograting assay, the sample is contacted with a support having a biograting (essentially non-light disturbing diffraction grating pattern) of active endometriosis antigen thereon. The light diffracted by the diffraction grating formed by binding of the antibodies in the sample is then determined.

Immunoassay reagents and kits including reagents for use with the methods of this invention are claimed. The immunoassay reagents include labeled EA 101A, EA 101B and EA 101C antigens. They also include insoluble supports to which are adhered, EA 101A, EA 101B and EA 101C antigens, or mixtures of one or more of the antigens. Kits include labeled anti-(endometriosis) antibodies, labeled or insolubly supported endometriosis antigens such as EA 101A, EA 101B, EA 101C antigens, mixtures of the endometriosis antigens, and/or labeled endometriosis antiidiotype antibodies. Labels include luminescent substances such as phosphors or fluorogens, bioluminescent substances, chemiluminescent substances, radioactive substances, enzymes, chromophores, pigments, spin labels, and metal containing substances. Determination of the label is by a means appropriate to the particular label used.

The present invention also provides an antibody which binds preferentially with endometriosis antigen. The antibody may be conjugated to a labeling moiety.

"Anti-(endometriosis) antibodies" are antibodies which are directed to an endometriosis marker, and which may be used for diagnosis of endometriosis. The term "anti(endometriosis) antibody" is defined to include monoclonal and polyclonal antibodies derived from any species which bind preferentially with endometriosis antigen and which are not substantially cross-reactive with normal endometrial tissue.

The term "antibody" as used herein is defined to include antibodies of classes IgG, IgM, IgA, IgD, and IgE, and fragments, half-antibodies, and hybrid derivatives of antibodies, including Fab, and F(ab$'$)$_2$ fragments of antibodies. Antibodies may be polyclonal or monoclonal. Generally, monoclonal antibodies are preferred for use in the methods of this invention.

The term "endometriosis antigen" is defined herein to be an endomtriosis epitope, or tissue fragment including the epitope, which is characteristic of endometriosis tissue but which is not characteristioc of normal (i.e., non-endometriosis) endometrial tissues. An example is an epitope or protein which binds preferentially with MS2B6 hMab. Included in this definition are heterogeneous tissue fragments; purified homogeneous protein fragment compositions; MS2B6 binding proteins or peptides from cell lines; and the isolated epitope, free from tissue components which are not essential for the binding properties of the epitope.

The term "endometriosis marker" is defined to be the epitope or epitopes present in endometriosis antigen which bind with anti-(endometriosis) antibody.

The term "endometriosis antiidiotype antibody", as used herein, is defined to be an antiidiotype antibody which will specifically bind with the binding site of the anti-(endometriosis) antibody and will compete with the endometriosis antigen for binding with the anti-(endometriosis) antibody.

The term "immunoassay", as used herein, is defined to mean a method using a preferential binding property of an endometriosis antibody with its endometriosis antigen binding partner, endometriosis antiidiotype antibody with its endometriosis antibody binding partner, or endometriosis antigen with its endometriosis antibody. Included within the scope of this invention are all immunoassay methods including this step, including but not limited to sandwich, competition, agglutination, precipitation, transistor bridge probe, particle sorting, light disturbing, light scattering, and ultrasonic probe immunoassays, for example. Appropriate immunoassays may use, as labels, radioisotopes, enzymes, or fluorogenic, chromogenic, or chemiluminescent substances.

The term "biograting", as used herein, is defined to be a substantially flat surface having a coating thereon and having substantially uniform light scattering properties. The coating comprises a diffraction grating pattern of alternating zones of an active and deactivated binding agent. The zones form a diffraction grating when the active binding agent binds with its opposite member of the binding pair. In the absence of such binding, no significant light diffraction occurs, that is, light energy detected at the diffraction angles is at a minimum value, approaching zero. When the binding occurs, the accumulation of bound material in the patterns of a diffraction grating creates a light disturbing grating, and light detected at the light diffraction angles increases to a large value which correlates to the presence and quantity of the binding partner (analyte) in the sample.

The term "light disturbing", as used herein, is defined to include all ways in which light is affected including light absorbing, reflecting, scattering, refracting and phase changing.

The term "diffraction grating", as used herein, is defined to include gratings which are formed in one or more immunological steps. For the method of this invention, the diffraction grating is formed directly by the conjugation of the non-light disturbing binding reagent on the insoluble surface with an analyte to form a light disturbing grating. Types of gratings formed in the process of this invention include reflection amplitude gratings, transmission amplitude gratings, reflection phase gratings, and transmission phase gratings. In reflection amplitude gratings, light is reflected from the grating, and the amplitude of the reflected light is modulated by the spatially variable reflection of the grating. In transmission amplitude gratings, light is transmitted through the grating, and the amplitude of the transmitted light is modulated by the spatially variable transmission of the grating. In the reflection phase grating, the light is reflected from

3

the grating, and the phase of the reflected light is modulated by the spatially variable refractive index of the grating. In the transmission phase gratings, light is transmitted through the grating, and the phase of the transmitted light is modulated by the spatially variable refractive index of the grating. In the method of this invention, the diffraction grating may function as one or more of these types of gratings concurrently, and all of these grating types are included within the diffraction gratings made in the method of this invention.

The diagnostic methods of this invention involve binding endometriosis antigen or anti-(endometriosis) antibody with an appropriate labeled reagent. Antibodies can be obtained from the ATCC HB 9765 hybridoma, or they can be prepared from endometriosis antigen.

Endometriosis antigen can be isolated by affinity chromatography of extracts of endometriosis tissue which has been extracted with neutral detergent, for example, using MS2B6 hMAb bound to conventional affinity column materials. The treatment of the tissue extract with the column and the elution of the endometriosis antigen from the column can be effected by conventional procedures. Suitable procedures for extracting and purifying proteins are described by H. Davis et al, Canc.Res. 46:6143-6148 (1986); V. Johnson et al, Canc.Res. 46:850-857 (1986); and E. Friedman et al, Canc.Res. 46:5189-5194 (1986); the entire contents of each of the above publications being hereby incorporated by reference.

Endometrial antigens can be obtained from tissue culture media such as cultures of epithelial carcinoma cell lines such as endometrial carcinoma AN3CA (ATCC -HTB 111) * , Breast carcinoma BT 20 (ATCC - HTB 19) * ; MCF-7 (ATCC-HTB 22) * , ovarian carcinoma No. 2774 and CaOV$_3$ (ATCC - HTB 75) * .

They can also be isolated from the sera of patients with endometriosis using, for example, using MS2B6 hMAb bound to conventional affinity column materials as described above for treatment of tissue digests.

Polyclonal antibodies can be prepared by conventional procedures, with any mammal used for polyclonal antibody production. Generally a rabbit, guinea pig or goat is adequate. In producing the antibody, a predetermined amount of endometriosis antigen is diluted with a physiological saline solution in a suitable concentration. This diluted solution is further diluted by mixing it with a complete Freund's adjuvant to prepare an emulsion. The emulsion is then administered to the mammal. For example, the suspension can be administered by intraperitoneal, intramuscular or subcutaneous routes to a rabbit in an amount of 0.05 mg per kilogram of body weight, to a maximum, non-lethal dose which might be as high as 5 mg of the antigen in every administration, and the administration can be continued every other week for 2 to 10 months. Blood is removed from the mammal when the antibody titer is sufficiently elevated, generally one to two weeks after the last challenge administration of the suspension. The blood taken from the animal is treated by centrifugal separation to separate the serum containing the antibody.

The polyclonal antibodies can also be prepared by covalent bonding of the isolated epitope or a polypeptide sequence including the epitope with an antigenic protein by conventional procedures which do not alter the epitope. Suitable coupling procedures are described by U.S. Patents 3,654,090, 4,214,048, 4,289,747, 4,302,438, 4,312,943, 4,376,110, and RE-31,006, and the references cited therein, for example.

The polyclonal antibody serum is then affinity purified using conventional affinity chromatography techniques such as those described by Mishell and Shilgi in SELECTED METHODS IN CELLULAR IMMUNOLOGY. San Francisco: Freeman (1980), the entire contents of which are hereby incorporated by reference. Suitable absorbents for use in affinity chromatography include cross-linked agarose and cross-linked polyacrylamides to which the MS2B6 IgM antibody is covalently bonded.

In these procedures, the antibody solution can be applied to the column in a phosphate buffered saline solution, and the antibodies can be eluted with a 2.5 M NaSCN solution, pH 8.0. Antibody concentration, if desired, can be achieved by negative pressure dialysis or ultrafiltration. The antibody solution is stable at temperature of 4° C or less.

Non-human monoclonal antibodies of this invention can be made from the endometriosis antigen or epitope coupled with an antigenic protein, purified as described above, and prepared by conventional procedures, generally following the method of Kohler and Milstein, Nature. 256:495-497 (1975). More recent developments are reviewed in J. Goding, MONOCLONAL ANTIBODIES: PRINCIPLES AND PRACTICE. New York: Academic Press pp 56-97 (1983). In general, the hybridoma is prepared by immunizing mice or rats with the antigen. While female A/J mice (H-2a haplotype, Jackson Laboratories, Bar Harbor, ME) are preferred, it is contemplated that other mice or rat strains can be used. The immunization schedule and concentration of antigen in the suspension should be such as to produce useful quantities of suitably primed splenocytes and/or lymphocytes. The suspended spleen cells are fused with mouse or rat myeloma cells from a suitable cell line by the use of a suitable fusion promoter. This can be, for example, Sendai

*Other suitable cell lines are widely available and the above are given by way of example.

Virus, polyethylene glycol or an electrical field. Many mouse myeloma cell lines are known and available, generally from members of the academic community and various deposit banks such as the American Type Culture Collection, Rockville, Md. Balb/C myeloma cells lines are preferred. The myeloma cell line used should preferably be medium sensitive so that unfused myeloma cells will not survive in a selective medium, while hybrids will survive. The most common class is 8-azaguanine resistant cell lines, which lack the enzyme hypoxanthine guanine phosphoribosyl transferase and hence will not be supported by HAT (hypoxanthine, aminopterin, and thymidine) medium. It is also generally preferred that the myeloma cell line used be of the so-called "non-secreting" type, in that it does not produce any antibody, although secreting types may be used. While the preferred fusion promoter is polyethylene glycol having an average molecular weight from about 1000 to 4000 (commercially available as PEG 1000, etc.), other fusion promoters known in the art can be used.

The supernatant in each container (well) containing a hybridoma is then examined for the presence of antibody which binds selectively with endometriosis. Procedures suitable for screening are described by J. Goding (supra, pp 72-84). One suitable method involves a competition between an anti-mouse immunoglobulin bound to an insoluble support such as a microtiter tray well and a mixture of labeled antigen and culture supernatant, the amount of label bound to the insoluble support being read to determine the binding of supernatant with the antibodies in the culture supernatant. Another suitable procedure comprises the application of the culture supernatant in a dot to a layer of nitrocellulose paper to which the selected isotype is adhered, rinsing the paper, contacting the paper with a chromogen labeled antibody or fluorescent labeled antibody which will bind to the Fc portion of any antibody bound to paper, rinsing the paper to remove unbound labeled antibody, and examining the paper to determine if bound chromogen or fluorogen is evident where the dot was applied. Automatic tray readers can be used to quickly identify the wells having hybridomas yielding antibodies which bind to the proteins adhered to the insoluble surface.

After the desired hybridomas have been selected and cloned, the resultant antibody can be produced by in vitro culturing in a suitable medium, followed by recovery of the antibody from the supernatant. Alternatively, the hybridoma can be injected into mice, preferably syngeneic or semisyngeneic mice. The hybridoma will cause formation of antibody producing tumors after a suitable incubation time. These will produce a high concentration of the desired antibody (about 5-20 mg/mL) in the bloodstream and peritoneal exudate (ascites) of the host mouse. Although the host mice will also have normal antibodies in their blood and ascites, the concentration of the normal antibodies will be only about 5 percent of the concentration of the desired monoclonal antibody.

Human monoclonal antibodies can be used in the methods herein. A human-human hybridoma, MS2B6, has been prepared according to the methods of L.H. Smith et al, *J.Immunol.Meth.* **105**:263-273 (1987), the disclosure of which, and the references cited therein, being hereby incorporated by reference in their entireties. MS2B6 IgM antibody is produced from this hybridoma in serum-free medium and purified by conventional procedures such as are described by J. Goding (supra, pp 98-118). Other methods for preparation of human-human monoclonal antibodies are shown in R. Cote et al, *Proc.Natl.Acad.Sci., U.S.A.* **83**:2959-2963 (1986); M. Glassy, *Canc.Res.* **47**:5181-5188 (1987); Borup-Christensen et al, *Int.J.Canc.* **37**:683-688 (1986); and M. Haspel et al, *Canc.Res.* **45**:3951-3961 (1985); the disclosures of each of which and of the literature cited therein being incorporated herein by reference in their entireties.

Antiidiotype antibodies can be prepared from the endometriosis antigen as described by P. Chen et al, *J.Exp.Med.* **162**:487-500 (1985), and in PTC Application No. 8502909 and EP Application No. 141783. Monoclonal antiidiotype antibodies to endometriosis antigen can also be prepared by the procedures described in U.S. Patent 4,513,088.

Polyclonal antiidiotype antibodies can be prepared, for example, by passing the animal serum obtained by immunizing with endometriosis antigen through a column to which MS2B6 hMAb antibody has been covalently bonded by conventional procedures well known in the art. After rinsing to remove residual serum, the column is eluted with a suitable buffer such as sodium acetate buffer to remove the antiidiotype antibody which has been separated, and the eluate is purified by dialysis to remove small molecules in a conventional manner.

Monoclonal antiidiotype antibodies can be prepared by screening the clones from the hybridized spleen cells of mice immunized with purified endometriosis antigen, or with anti-endometriosis antibodies such as MS2B6 hMAb antibodies, and screening the wells for clones producing antibodies binding with the MS2B6 hMAb binding site. MS2B6 hMAb bound to a microwell is incubated with hybridoma supernatant for a time sufficient to permit antibody-antigen binding to occur, the supernatant is removed from the well, and the well is rinsed. The well is then incubated with enzyme labeled goat or rabbit anti-Ig or anti-IgM antibody for a time sufficient to permit antibody-antigen binding to occur, and the residue is removed from the well by rinsing. A substrate yielding a chromophore or fluorophore in the presence of the enzyme is then added to the well, and the presence the chromophore or fluorophore is used as a criterion for selecting clones. Control wells must also be tested with irrelevant IgM. Only antibodies having activity against MS2B6 hMAb and not against irrelevant human MAb are antiidiotype candidates. Preferably, they should be selected to inhibit or compete with the binding.of endometriosis antigen to MS2B6.

The antibodies of this invention can be coupled with a variety of moieties having one or more distinctive properties which are useful for diagnostic methods. The distinctive property can be any detectable label which can be used, directly or indirectly, to form an image. Antibodies can be radiolabeled, or labeled with enzymes, luminescent substances such as phosphors or fluorogens, bioluminescent substances, chemiluminescent substances, chromophores, pigments, spin labels, and metal containing substances, for example.

Radiolabeled antibodies of this invention can be used for in vitro diagnostic tests. The specific activity of a tagged antibody depends upon the half-life, isotopic purity of the radioactive label and how the label is incorporated into the antibody. Table A lists some commonly used isotopes, their specific activities, and half-lives. For immunoassay tests in general, the higher the specific activity, the better the sensitivity.

TABLE A

| Isotope | Specific Activity of Pure Isotope (Curies/mole) | Half-Life |
|---------|--------------------------------------------------|-----------|
| $^{14}C$ | $6.25 \times 10^1$ | 5720 years |
| $^3H$ | $2.91 \times 10^4$ | 12.5 years |
| $^{35}S$ | $1.50 \times 10^6$ | 87 days |
| $^{125}I$ | $2.18 \times 10^6$ | 60 days |
| $^{32}P$ | $3.16 \times 10^6$ | 14.3 days |
| $^{131}I$ | $1.62 \times 10^7$ | 8.1 days |

Procedures for labeling antibodies with radioactive isotopes listed in Table A are generally known in the art. Tritium labeling procedures are described in U.S. Patent 4,302,438, for example. Iodinating, tritium labeling and $^{35}S$ labeling procedures especially adapted for murine monoclonal antibodies are described by J. Goding (supra, pp 124-126) and the references cited therein. Other procedures for iodinating antibodies are described by Hunter and Greenwood, *Nature.* **144**:945 (1962); David et al, *Biochem.* **13**:1014-1021 (1974); and in U.S. Patents 3,867,517 and 4,376,110.

Radiolabeling elements which are especially useful in diagnosis include $^{125}I$, $^{131}I$, $^{111}In$, and $^{99m}Tc$, for example. Procedures for iodinating antibodies are described by F. Greenwood et al, *Biochem.J.* **89**:114-123 (1963); J. Marchalonis, *Biochem.J.* **113**:299-305 (1969); and M. Morrison et al, *Immunochem.* **8**:289-297 (1971). Procedures for $^{99m}Tc$-labeling are described by B. Rhodes et al. in S. Burchiel, et al (eds.), TUMOR IMAGING: THE RADIOIMMUNOCHEMICAL DETECTION OF CANCER. New York: Masson 111-123 (1982), and the references cited therein. Procedures suitable for $^{111}In$-labeling antibodies are described by D. Hnatowich et al, in *J.Immunol.Meth.* **65**:147-157 (1983), in *Science.* **220**:613-615 (1983), and in *J.App.Rad.* **35**:554 557 (1984); and by R.G. Buckley et al, in *F.E.B.S.* **166**:202-204 (1984).

Antibodies labeled with enzymes are useful for in vitro diagnostic tests. Examples of suitable systems, coupling procedures and substrate reactions therewith are disclosed in U.S. Patents 3,654,090, 4,214,048, 4,289,747, 4,302,438, 4,312,943, 4,376,110, and RE-31,006, and the references cited therein, for example. Examples of other suitable systems are described by Pesce et al, *Clin.Chem.* **20**:353-359 (1974) and G. Wisdom, *Clin.Chem.* **22**:1243 (1976).

A list of suitable enzyme classes and specific examples for each class are shown in Table B.

TABLE B

| Class | Enzyme Example |
|---|---|
| Hydrolases | Amylases |
| Nucleases | Polynucleotidase |
| Amidases | Arginase |
| Purine deaminases | Adenase |
| Peptidases | Aminopolypeptidase |
| Proteinases | Pepsin |
| Esterases | Lipases |
| Iron Enzymes | Catalase |
| Copper Enzymes | Tyrosinases |
| Enzymes containing Coenzymes | Alcohol dehydrogenase |
| Enzymes reducing cytochrome | Succinic dehydrogenase |
| Yellow enzymes | Diaphorase |
| Mutases | Glyoxalase |
| Desmolases | Aldolase |
| Oxidases | Glucose oxidase |
| | Horseradish peroxidase |
| Phosphatases | Alkaline Phosphatases |
| | Acid Phosphatases |
| Dehydrogenases | G6PDH (Glucose 6-phospho-dehydrogenase) |
| β-galactosidase | |
| Phosphorylases | |
| Hexokinases | |

A list of suitable enzymes is given in Hawk, et al. PRACTICAL PHYSIOLOGICAL CHEMISTRY, New York: McGraw-Hill pp 306-397 (1954).

Fluorogenic and chromogenic enzymes (enzymes in the presence of which a selected substrate will produce a fluorescent or chromogenic product) are useful labeling moieties. Methods for selectively conjugating enzymes to antibodies without impairing the ability of the antibody to bind with antigen and for conjugating enzymes to proteinaceous reagents are well known in the art. Suitable enzymes and procedures for coupling them to antibodies are described by I. Chibata in IMMOBILIZED ENZYMES. Halsted Press: New York (1978); A. Cuatrecasas, *J.Bio.Chem.* **245**:3059 (1970); M. Wilson et al, INTERNATIONAL CONFERENCE IN IMMUNOFLUORESCENCE AND RELATED STAINING TECHNIQUES. W. Knapp et al, editors. Amsterdam: Elsevier pp 215-244 (1978); M. Sullivan et al, *Ann.Clin.Biochem.* **16**:221-240 (1979); H. Nygren et al, *Med.Biol.* **57**:187-191 (1979); D. Gadkari et al, *J.Virol.Meth.* **10**:215-224 (1985); P. Tijssen et al, *Anal.Biochem.* **136**:451-457 (1984): J. Tsuruta et al, *J.Histochem.Cytochem.* **33**:767-777 (1985); E. Ishikawa,*J.Immunoassay*, **4**:209-327 (1983); and in U.S. Patent 4,190,496, for example, the entire contents of the above references being incorporated by reference in their entireties.

The preferred enzymes and suitable substrates corresponding thereto include horseradish peroxidase, for which suitable substrates are *o*-phenylenediamine, *m*-phenylenediamine, *o*-dianisidine, and 4-chloro-α-napthol; β-galactosidase, for which suitable substrates are 4-methylumbelliferyl-β-D-galactoside, *p*-nitrophenyl-β-D-galactose, *p*-nitrophenol, *o*-nitrophenyl-β-D-galactose, and *o*-nitrophenol, for example; and alkaline phosphatase, for which suitable substrates are *p*-nitrophenylphosphate, indoxyl phosphate, and 5-bromo-3-chloroindoxyl phosphate, for example.

Examples of suitable procedures for enzyme labeling the antibody include the use of carbodiimides, dialdehydes, and gluteraldehyde bifunctional coupling reagents. Linkage of enzymes through amine groups can be achieved by treating the proteins with thionyl chloride, N-hydroxysuccinimide or similar reagents in an anhydrous solvent such as dimethylformamide, dioxane, dimethylsulfoxide, tetrahydrofuran, or the like. Alternative coupling agents include carbodiimides such as 1-ethyl-3-(3-(N,N'-dimethylamino)propyl)-carbodiimide, 1-cyclohexyl-3-(2-morpholinoethyl)carbodiimide methyl-*p*-toluenesulfonate, succinimidyl 4-(N-maleimidoethyl)-cyclohexane-1-carboxylate, and succinimidyl 3-(2-pyridyldithio)-propionate, for example.

The carbohydrate moiety of an enzyme can also be oxidized to an aldehyde and reacted with lysyl amino groups of immunoglobulins to form a Schiff's base. Reduction with sodium borohydride effects a stable linkage of enzyme and antibody. Horseradish peroxidase can be efficiently linked to immunoglobulins by the method of M. Wilson et al, INTERNATIONAL CONFERENCE IN IMMUNOFLUORESCENCE AND RELATED STAINING TECHNIQUES. W. Knapp et al, editors. Amsterdam: Elsevier pp 215-244 (1978).

Fluorophore and chromophore labeled antibodies can be prepared from standard moieties known in the art. Since antibodies and other proteins. absorb light having wavelengths up to about 310 nm, the fluorescent moieties should be selected to have substantial absorption at wavelengths above 310 nm and preferably above 400 nm. A variety of suitable fluorescers and chromophores are described by Stryer, Science. 162:526 (1968) and L. Brand et al, Ann.Rev.Biochem. 41:843-868 (1972). The antibodies can be labeled with fluorescent chromophore groups by conventional procedures such as those disclosed in U.S. Patents 3,940,475, 4,289,747 and 4,376,110, for example.

One group of fluorescers having a number of the desirable properties described above are the xanthene dyes, which include the fluoresceins derived from 3,6-dihydroxy-9-phenylxanthhydrol and resamines and rhodamines derived from 3,6-diamino-9-phenylxanthydrol and lissanime rhodamine B. The rhodamine and fluorescein derivatives of 9-o-carboxyphenylxanthhydrol have a 9-o-carboxyphenyl group. Fluorescein compounds having reactive coupling groups such as amino and isothiocyanate groups such as fluorescein isothiocyanate and fluorescamine are readily available. Another group of fluorescent compounds are the naphthylamines, having an amino group in the $\alpha$ or $\beta$ position. Antibodies can be labeled with fluorochromes or chromophores by the procedures described by J. Goding (supra, pp 208-249). A preferred embodiment of the methods herein use avidin-labeled and biotin-labeled antibodies and reagents, for enhancement of the labeling signal.

The labeled reagents can be used to determine the presence of endometriosis antigen in a tissue sample using immunohistopathological staining tissues. Tissue samples can be obtained by laproscopy or biopsy, for example. Tissues or cell samples are prepared and mounted on slides by standard procedures such as those described by L. Koss, DIAGNOSTIC CYTOLOGY AND ITS HISTOPATHOLOGIC BASIS. Philadelphia: Lippincott (1979); L. Luna, MANUAL OF HISTOLOGIC STAINING METHOD OF THE ARMED FORCES INSTITUTE OF PATHOLOGY, 3rd ed. New York: McGraw-Hill (1968); and M. Borowitz et al, J.Histochem.Cytochem. 30:171-174 (1982). In general, the tissue is quick frozen and frozen sections are prepared and mounted on slides.

In staining procedures, the anti-(endometriosis) antibody is applied to the sample being tested. Anti-(endometriosis) antibody can be applied to a tissue section or cell smear on the slide in an aqueous solution. The solution preferably contains suitable salts and buffers to preserve the reactants and facilitate the binding reaction. For example, the solution can contain protein such as bovine serum albumin (BSA), phosphate buffer solution (PBS), and a mild surfactant. The buffered solution can contain from 1 to 100 $\mu$g/mL, and preferably contains from 10 to 25 $\mu$g/mL, of the primary antibody in an aqueous phosphate buffered solution having a phosphate molarity of from 0.005 to 0.1 and a pH of from 6.0 to 8.0. A phosphate molarity of from .01 to 0.5, and a pH of from 7.2 to 7.6 are preferred. The primary antibody solution is maintained in contact with the cell smear until binding with antigen can occur. The incubation time is temperature dependent. At temperatures of from 18 to 40 $^{\circ}$C, incubation times of from 30 to 180 minutes can be used. At room temperature, incubations times can be from 1 to 60 minutes and preferably from 5 to 30 minutes.

The excess primary antibody solution is then removed, and the slide is rinsed with a suitable rinse solution. Rinse solutions can be aqueous phosphate buffered solutions having a phosphate molarity of from 0.1 to 0.5, a pH of from 6 to 8.

If the primary antibody is labeled, the label is directly or indirectly determined. In one preferred method of this invention, a biotin-labeled primary anti-(endometriosis) antibody is applied. The tissue is then contacted with an avidin-labeled biotin-complex. For example, if the primary antibody is labeled with biotin, the preferred avidin-labeled biotin complex is prepared by mixing a large molar excess of avidin with a biotinylated enzyme. The cross-binding of the avidin and biotin complexes causes a multiplication of the labeling signal for each bound antibody. This signal enhancement increases the sensitivity of the assay. Commercially available avidin-biotin labeling systems include the Vectastain\ systems, such as Vectastain\ ABC, from Vector Laboratories, Burlingame, CA.

The avidin or biotin can be labeled with any conventional label such as a luminescent substance such as a phosphor or fluorogen, a bioluminescent substance, a chemiluminescent substance, a radioactive substance, an enzyme, chromophor, pigment, spin label, or metal containing substance. These labels are covalently bonded to the avidin or biotin by conventional procedures appropriate to the chemical groups on the label. Procedures for covalently bonding radioactive isotopes, fluorophors and enzymes to avidin or biotin can be the same procedures described above for binding the respective labels to anti-(endometriosis) antibodies, for example.

The slide is examined by procedures appropriate for the particular avidin-biotin complex label employed to determine antigen-antibody binding in the sample. These procedures are conventional. For example, if a radioactive label is employed, the slide can be examined with a Geiger counter to determine the level of residual radioactivity on the slide. Alternatively, if the label is a phosphor or a fluorogen, it can be examined under a fluorescent microscope. If the label is a chromophor or a pigment, the slide can be examined under a microscope using ordinary light. The cells to which anti-(endometriosis) antibody is significantly bound are labeled, showing that they are endometriosis tissue.

In the preferred embodiment of this invention, the biotin-avidin complex has an enzyme label. Suitable enzymes and substrate systems are described in U.S. Patents 4,190,496 and 4,208,479, and in Hawk, et al, PRACTICAL PHYSIOLOGICAL CHEMISTRY. New York:McGraw-Hill pp 306-307 (1954), which are hereby incorporated by reference. The system selected is designed to provide the distinguishing characteristics desired. Preferred systems employ oxidoreductases such as horseradish peroxidase and a substrate such as diaminobenzidine. The horseradish peroxidase and diaminobenzidine provide a pronounced pigmentation or stain of the endometriosis, which contrasts strongly with available counter-stains. Any other enzyme-substrate combinations providing a basis for clearly distinguishing the endometriosis and normal cells can be used.

Biotin and avidin can be optionally interchanged or mutually substituted in the method and reagents of this invention. Corresponding adjustments in molar ratios between biotin and avidin derivatives are then required. Avidin-biotin signal enhancement has been explained in detail in reference to tissue staining with a biotin-labeled primary antibody. It will be obvious to those skilled in the art that modifications of the preferred avidin-biotin label enhancement system can be made, and that the avidin-biotin label enhancement system can be used for immunohistochemical staining using labeled secondary antibodies, for fluid sample assays, for assays in which reagents are bonded to an insoluble support, for competition assays, and for sandwich assays. Such obvious modifications are within the spirit and scope of the invention herein.

In an alternative immunohistochemical assay method, the primary antibody is an anti-(endometriosis) non-human antibody and is unlabeled. The primary antibody is bound to the antigens present in the sample, as above, and excess primary antibody is removed. A secondary antibody is labeled with biotin. The secondary antibody binds selectively with the class of antibodies from which the primary antibody is selected. This secondary antibody is applied to the tissue in a suitable aqueous solution for a time sufficient to permit binding between the secondary antibody and any primary antibody bound to cell antigens. In general, the secondary antibody is preferably a monoclonal antibody which binds to the class of immunoglobulin to which the primary antibody is a member, for example to the Fc portion of the primary antibody. For primary antibodies of the IgM, IgG$_1$ , IgG$_{2a}$, IgG$_{2b}$, IgA, and other classes, for example, secondary antibodies binding to the respective Fc portions or the primary antibodies are selected.

The biotin is covalently bonded to the secondary antibody by conventional procedures such as those described above for binding biotin to the primary antibody, with a large molar excess of biotin to antibody, preferably with a molar ratio of at least 100:1 biotin to antibody. The biotin labeled secondary antibody is applied to the tissue section in an aqueous solution which is preferably the phosphate buffered solution described above as the vehicle for the original primary antibody. The solution is contacted or incubated with the cells on the slide for a time sufficient to permit binding between the secondary antibody and the primary antibody, if any is present, to occur. The preferred incubation times and temperatures correspond to those described above with respect to the treatment of the tissue sample or cell smear with the primary antibody solution in the first step. Following incubation, the excess secondary antibody solution is removed, and the slide is rinsed with a suitable rinse solution, such as that described above. The preferred avidin-labeled biotin complex can be used to enhance the signal, as described above.

The reagents of this invention can be used in sandwich and competition immunoassays to detect substances having the endometriosis antigen epitope in the blood or other body fluid, the presence of the substances in the fluid being indicative of endometriosis.

A competition immunoassay embodiment of this invention is a method for determining the presence and amount of endometriosis antigen in a body fluid, for example blood plasma. In the method, the sample is mixed with a predetermined amount of labeled endometriosis antigen or labeled endometriosis antiidiotype antibody, and the mixture is contacted with an insoluble support to which anti-(endometriosis) antibody is adhered. The amount of anti-(endometriosis) antibody is insufficient to bind all of the sample analyte and the labeled endometriosis antigen or endometriosis antiidiotype antibody. The mixture is contacted with the insoluble support for a time sufficient to permit antigen-antibody binding to occur, and the sample is removed from the support. The label remaining bound on the insoluble support is then measured. The label can be a physically detectable label which can be measured directly on the insoluble support. Alternatively, the label can be a moiety which upon subsequent treatment yields a physically detectable label. For example, if the label is an enzyme label, the insoluble support can be contacted with a substrate for the enzyme which will yield a measurable fluorophore or chromophore. The amount of label measured is an inverse function of the amount of the endometriosis antibody in the body fluid.

A preferred immunoassay is a sandwich immunoassay embodiment of this invention. The method determines the presence and amount of anti-(endometriosis) antibody in a body fluid sample, for example a blood sample. In this method, the body fluid is contacted with an insoluble support to which endometriosis antigen or endometriosis antiidiotype antibody is adhered, for a time sufficient to permit antigen-antibody binding to occur, and the sample is removed from the support. The insoluble support is then contacted with an anti-Ig antibody (i.e., a secondary antibody) to bind with any sample antibody bound to the insoluble support. The presence of secondary antibody on the insoluble support is then determined. The secondary antibody can have a physically detectable label which can be measured directly on the insoluble support. Alternatively, the secondary antibody can be unlabeled, and the secondary antibody can be determined by contacting the insoluble support with a labeled antibody which binds selectively with the secondary antibody (i.e., a tertiary antibody), removing unbound labeled tertiary antibody from the support, and determining the presence of the label on the insoluble support. Avidin-biotin signal enhancement such as that described previously can be used to amplify the label.

An alternate sandwich immunoassay embodiment of this invention determines endometriosis antigen or endometrio-sis antiidiotype antibody in a body fluid sample, for example a blood sample. In this method, the body fluid is contacted with an insoluble support to which anti-(endometriosis) antibody is adhered, for a time sufficient to permit antigen-antibody binding to occur, and the sample is removed from the support. The insoluble support is then contacted with an anti-(endometriosis) antibody (i.e., a secondary antibody) to bind with any sample antigen or antiidiotype antibody bound to the insoluble support. The presence of secondary antibody on the insoluble support is then determined. The secondary antibody can have a physically detectable label which can be measured directly on the insoluble support. Alternatively, the secondary antibody can be unlabeled, and the secondary antibody can be determined by contacting the insoluble support with a labeled antibody which binds selectively with the secondary antibody (i.e., a tertiary antibody), removing unbound labeled tertiary antibody from the support, and determining the presence of the label on the insoluble support. Avidin-biotin signal enhancement such as that described previously can be used to amplify the label.

A particularly advantageous immunoassay of this invention uses a biograting. Biograting methods, materials and apparatus are described in U.S. Patents 4,647,544 and 4,876,208, the entire contents of which are hereby incorporated by reference. In this method, the sample is contacted with an endometriosis antigen biograting for a sufficient time to permit the endometriosis antigen to bind with endometriosis antibody in the sample. After the sample is removed from the biograting surface, the light diffracted by the grating formed by the binding reaction is detected and measured.

The antibody and antigen reagents can be bonded to an insoluble support by conventional processes. Procedures for binding of antibodies to insoluble supports are described in U.S. Patents 3,551,555, 3,553,310, 4,048,298 and RE-29,474, and in P. Tijssen, LABORATORY TECHNIQUES IN BIOCHEMISTRY AND MOLECULAR BIOLOGY: PRACTICE AND THEORIES OF ENZYME IMMUNOASSAYS. New York: Elsevier (1985), for example. Procedures for binding of antibodies to polystyrene by adsorption are described in U.S. Patents 3,646,346 and 4,092,408, for example. These same procedures are suitable for binding proteinaceous antigens to insoluble supports.

A variety of materials can be used as the insoluble support, the primary consideration being the binding of the antibody or antigen to the surface, the absence of interference with the reagent binding reaction or with other reactions which can be employed to determine the presence and extent of the binding reaction. Organic and inorganic polymers, both natural and synthetic, can be used as the insoluble support. Examples of suitable polymers include polyethylene, polypropylene, polybutylene, poly(4-methylbutylene), butyl rubber, silastic polymers, polyesters, polyamides, cellulose and cellulose derivatives (such as cellulose acetate, nitrocellulose and the like), acrylates, methacrylates, vinyl polymers (such as polyvinyl acetate, polyvinyl chloride, polyvinylidene chloride, polyvinyl fluoride, and the like), polystyrene and styrene graft copolymers, rayon, nylon, polyvinylbutyrate, polyformaldehyde, etc. Other materials which can be used as the insoluble support are the latexes of the above polymers, silica gel, silicon wafers, glass, paper, insoluble protein, metals, metalloids, metal oxides, magnetic materials, semi-conductive materials, ceramics and the like. In addition are included substances which form gels, e.g. proteins such as gelatins, lipopolysaccharides, silicates, agarose, polyacrylamides or polymers which form several aqueous phases such as dextrans, polyalkylene glycols (alkylene with 2 to 3 carbon atoms) or surfactants, e.g. amphophilic compounds such as phospholipids, long chain (12-24 carbon atoms) alkyl ammonium salts, and the like.

The preferred support comprises a nylon or nitrocellulose membrane. An alternate diagnostic support is made from polystyrene, styrene copolymers such as styrene-acrylonitrile copolymers, or polyolefins such as polyethylene or polypropylene, and acrylate and methacrylate polymers and copolymers. The antibody and antigen reagents can be bound to the insoluble support by adsorption, ionic bonding, van der Waals adsorption, electrostatic bonding, or other non-covalent bonding, or it can be bound to the insoluble support by covalent bonding. A particularly advantageous support for this procedure comprises a microtiter plate having a plurality of wells. The well surface or plastic cup inserts therein can constitute the antigen or antibody support. If the determination will require the use of fluorometric measurements, the microtiter plate or the well inserts are advantageously opaque to light so that excitation light applied to a well does not reach or influence contents of the surrounding wells.

Procedures for non-covalent bonding are described in U.S. Patent 4,528,267. Procedures for covalently bonding antibodies and antigens to insoluble supports are described by I. Chibata in IMMOBILIZED ENZYMES. Halsted Press: New York (1978) and A. Cuatrecasas, *J.Bio.Chem.* 245:3059 (1970), the entire contents of which are hereby incorporated by reference. The surface can be coated with a protein and coupled with the antibody or antigen using procedures described in U.S. Patent 4,210,418 using glutaraldehyde as a coupling agent, for example. In a still further procedure, the well can be coated with a layer having free isocyanate groups such as a polyether isocyanate, and application of the antibody or antigen in aqueous solution thereto effects the requisite bonding. In a still further procedure, the antibody or antigen can be coupled to a hydroxylated material by means of cyanogen bromide as described in U.S. Patent 3,720,760.

The insoluble supports are preferably "blocked" to reduce nonspecific binding. The choice of suitable blocking agents in determined by the type of insoluble support. For example, for polystyrene supports, suitable blocking agents include water-soluble non-immune animal proteins and polyamino acids. Suitable water-soluble non-immune animal proteins include bovine (BSA), human, rabbit, goat, sheep, and horse serum albumins; and other animal proteins such as fetal calf serum, ovalbumin, fibrinogen, thrombin, transferrin, glycoproteins, and the like. Suitable water-soluble polyamino acids include polymers of one or more amino acids such as lysine, glutamic acid, alanine, histidine, methionine, proline, and the like.

The same blocking agents can also be used for nylon and nitrocellulose supports. However, a preferred blocking agent for nitrocellulose or nylon membrane supports is nonfat milk or casein. An optimum blocking agent for these membrane supports is an aqueous solution containing 5 wt.% nonfat dried milk and non-ionic surfactants such as polyoxyethylene sorbitan derivatives and polyoxyethylene ethers.

For preparation of biogratings, any smooth surface having the requisite high binding affinity for binding reagent can be used in this process. For purposes of clear explanation and not by way of limitation, the process is described for a semiconductor wafer with a polished surface bearing a polysilicon coating. It should be understood that the same, equivalent, or similar procedures can be applied for preparing diffraction gratings designs with binding reagents with other high binding smooth surfaces. With the preferred insoluble supports of aluminum, silicon nitride, silicon dioxide, single crystalline silicon, and particular the polysilicon surfaces, the binding reagent can be applied by simple adsorption. In one procedure for non-covalent adhesion of binding reagent to the surface of an insoluble support, the endometriosis antibody or endometriosis antiidiotype antibody is applied to the surface of the support by immersing the surface in a buffered binding reagent solution for from 0.5 to 18 hours and preferable from 1 to 3 hours, at temperatures of from 4 to 40°C and preferable from 20 to 26°C. The polysilicon surface is then rinsed with a buffered saline solution and dried.

The concentration of binding reagent in the buffer solution is selected to provide the desired reagent density on the polysilicon surface. The binding reagent solution can contain from 0.01 to 100 mgrams/ml of the binding reagent and preferably contains from 10 μgrams/ml to 50 mgrams/ml of the binding reagent in a buffered solution having a pH of from 6.0 to 9.5 and preferably from 7.0 to 8.5.

A suitable rinse solution is an aqueous phosphate buffer solution such as is described in U.S. Patent 4,528,267 having a phosphate molarity of from 0.0001 to 0.20, a pH of from 6 to 8 and containing from 0.001 to 0.1 weight percent non-ionic surfactant and from 0.0001 to 5.0 weight percent of an animal serum albumin. Suitable non-ionic surfactants include polyoxyethylene ethers (BRIJ) such as lauryl, cetyl, oleyl, stearyl, and tridecyl polyoxyethylene ethers; polyoxyethylenesorbitans (TWEEN) such as polyoxyethylenesorbitan monolaurate, monopalmitate, monostearate, monoleate and trioleates; and other polyoxyethylene ethers (TRITON), for example. Preferred non-ionic surfactant are the polyoxyethylenesorbitans such as polyoxyethylenesorbitan monolaurate (TWEEN 20).

A mask is prepared by photographic methods conventional in semiconductor manufacturing. For example, a mask having a plurality of diffraction gratings with the desired line density and line widths can be prepared on a quartz glass or other UV-transparent plate through a photoresist process similar to photography. The dark lines of the mask correspond to active binding reagent areas desired on the ultimate surface.

The mask is placed over the polysilicon surface having a coating of binding reagent, and the surface is exposed to ultraviolet radiation until the binding capability of the portions of the binding reagent exposed to the radiation is substantially reduced or preferably eliminated. To manufacture a precision grating design, the radiation should form a sharp image on the coated surface. Penumbrae should be minimized. Preferably, the ultraviolet light passing through the mask is focused to a sharp image on the surface coating using conventional projection alignment techniques without contact with the coated surface.

The ultraviolet radiation exposure required to deactivate antibody coating exposed thereto is from 10 sec to 60 min and preferably from 1 to 5 min with ultraviolet radiation having a wavelength such as 254 nm and a power of from 8 to 14 mwatts per $cm^2$. Some adjustment in time of exposure and/or power may be necessary to deactivate the binding sites of other antibody binding agents.

The ultraviolet radiation exposure required to deactivate endometriosus antigen coating exposed thereto is from 1 to 60 min and preferably from 1 to 5 min with ultraviolet radiation having a wavelength such as 254 nm and a power of from 8 to 14 mwatts per $cm^2$. Some adjustment in time of exposure and/or power may be necessary to deactivate the binding sites of other antigen binding agents.

This treatment reduces or eliminates the binding properties of the binding reagent in exposed areas, leaving active binding reagent in a diffraction grating design corresponding to the opaque areas of the mask. The coated substrate containing areas having binding protein in a diffraction grating design is cut into smaller area chips, each chip having a size sufficient to perform a binding assay. These chips are then mounted on a suitable diagnostic support such as the dipstick.

The first step in the biograting binding assay method according to this invention comprises contacting a diffraction binding assay surface with the sample of blood, plasma, or serum of a patient, for a time sufficient to permit conjugation of active zones of endometriosis antiidiotype antibody or endometriosis antigen with endometriosis antibody in the sample. The diffraction binding assay surface is an insoluble surface with a light disturbing design of substantially non-light disturbing endometriosis antibody thereon.

The surface is then separated from the sample. The surface is preferably rinsed, for example with distilled or deionized water, and the excess water is removed.

The insoluble surface is then illuminated with light from a light source, and the diffraction of light by the insoluble surface is determined. The concentration of the endometriosis antibody present correlates with the diffraction intensity determined.

The strength of the light diffraction is measured with a suitable light diffraction instrument such as is illustrated hereinabove, adjusted to provide the angle of incidence, $\alpha$, within the ranges set forth in Table A. The relative strengths of the light diffracted is a function of the amount of primary binding reagent-analyte conjugate comprising the grating. By repeating the above procedure with a prepared series of solutions containing a range of different known concentrations of analyte therein, a standard curve functionally related to the strength of the diffracted light is obtained. By comparing the reading obtained with the sample containing the analyte with the curve obtained with solutions containing known concentrations of the analyte, the concentration of analyte in the sample can be determined. Comparing the strengths of the first, second, third, etc. order diffractions with each other and with the strength of the reflected light directly provides an indication of the degree of conjugation with the grating binding agent.

Reagents of this invention include endometriosis antigen and labeled derivatives thereof, insoluble supports having endometriosis antigen or antiidiotype antibody adhered thereto including microwells, membranes and, biogratings having active and deactivated zones of endometriosis antigen, endometriosis antiidiotype antibody.

The testing kits of this invention are combinations of reagents which are used together in the respective tests. The kits include but are not limited to the following combinations:

a) Endometriosis antigen or endometriosis antiidiotype antibody adhered to an insoluble support and a labeled secondary antibody;

b) Endometriosis antibody adhered to an insoluble support and labeled endometriosis antigen or endometriosis antiidiotype antibody;

c) Endometriosis antigen adhered to an insoluble support and labeled endometriosis antibody; and

d) Endometriosis antigen or antiidiotype antibody biograting and reference biograting.

This invention is further illustrated by the following specific but non-limiting examples. Temperatures are given in degrees Centigrade and percentages as weight percents unless otherwise specified. Procedures which have been previously carried out are presented in the past tense, and procedures which are being constructively reduced to practice in this application are presented in the present tense.

## EXAMPLE 1

EP 0 387 027 A2

*MS2B6 IgM Antibody*

Human hybridoma MS2B6 (ATCC HB 9765) is grown in serum-free medium (Iscoves with 1% Nutridoma-NS, Boehringer-Mannheim) and produced IgM at a rate of 32 Ig per $10^6$ cells per day. Supernatant fluids containing approximately 60 μg/mL IgM were concentrated 100-fold by ultrafiltration using a membrane with 300,000 dalton molecular weight cutoff (Amicon). The MS2B6 IgM was purified by HPIEC using a Model 1084B liquid chromatograph (Hewlett Packard) and a 0.75 x 7.5 cm TSK-DEAE-5PW column (Bio Rad). Buffer A consisted of 0.02 M Tris, 0.1 M sodium chloride, pH 8.5. Buffer B consisted of 0.02 M Tris, 0.6 M sodium chloride, pH 7.0. The column was equilibrated with 10 (v/v)% Buffer B in Buffer A, and the samples were applied. The column was eluted with 25 (v/v)% Buffer B in Buffer A. The eluted IgM was concentrated and stored frozen in liquid nitrogen. The purity of the final product was approximately 95% IgM by SDS-PAGE.

## EXAMPLE 2

*Endometriosis Antigen*

Endometriosis antigen is isolated by gel electrophoresis using the SDS-PAGE procedure. Larger quantities of the antigen can be obtained by affinity chromatography of extracts of endometriosis tissue which has been extracted with neutral detergent, using MS2B6 hMAb bound to conventional affinity column materials. The treatment of the tissue extract with the column and the elution of the the endometriosis antigen from the column can be effected by conventional procedures. Suitable procedures for extracting and purifying proteins are described by H. Davis et al, *Canc.Res.* **46**:6143-6148 (1986); V. Johnson et al, *Canc.Res.* **46**:850-857 (1986); and E. Friedman et al, *Canc.Res.* **46**:5189-5194 (1986); the entire contents of which are hereby incorporated by reference.

## EXAMPLE 3

*Polyclonal Anti-(Endometriosis) Antibodies*

The anti-(endometriosis) antibodies are elicited in rabbits using the immunization techniques and schedules described in the literature, e.g., Stollar, *Meth.Enzym.* **70**;70 (1980), immunizing the rabbits with the endometriosis antigen, prepared as described in Example 1. The antiserum is screened in a solid phase assay similar to that used for monoclonal antibodies, e.g., as described by Lange et al, *Clin.Exp.Immunol.* **25**:191 (1976) and Pisetsky et al, *J.Immunol.Meth.* **41**:187 (1981).

The IgG fraction of the antisera is purified further by affinity chromatography using CNBr-Sepharose 4B (Pharmacia Fine Chemicals) to which has been coupled endometriosis antigen. The method used for coupling is that recommended by the gel manufacturer, AFFINITY CHROMATOGRAPHY. Pharmacia Fine Chemicals, pp 15-18.

The column is equilibrated with from 2 to 3 volumes of buffer (0.01 M PBS, pH 7.2), and the anti-(endometriosis) antibody containing solution is then applied to the column. The absorbency of the eluate is monitored at 280 nm until protein no longer passes from the column. The column is then washed with 0.1 M glycine buffer, pH 2.5, to desorb the immunoaffinity bound anti-(endometriosis) antibody. Peak protein fractions are collected, pooled and dialyzed against 0.01 M PBS, pH 7.2, for 24-36 hr at 4° C with multiple buffer changes.

## EXAMPLE 4

### Monoclonal Endometriosis Antibodies

Using the purified endometriosis antigen obtained by the procedure of Example 2, mouse monoclonal antibodies to the endometriosis antigen are obtained using standard procedures of Galfre and Milstein, *Meth.Enzym.* **73**:1 (1981), using endometriosis antigen as the antigen for immunizing the mice. The monoclonal antibodies are screened using a modification of the techniques described in the literature, e.g., Lange et al, *Clin.Exp.Immunol.* **25**:191 (1976) and Pisetsky et al, *J.Immunol.Meth.* **41**:187 (1981).

Mouse monoclonal antibody is purified from ascites fluid or from hybridoma culture supernatants using Protein-A coupled Sepharose-4B (Pharmacia Fine Chemicals) according to the procedure of Tijsson, PRACTICE AND THEORY OF ENZYME IMMUNOASSAYS. Elsevier Science Publishers, pp 105-107 (1985).

## EXAMPLE 5

### Antiidiotype Endometriosis Antibodies

Polyclonal and monoclonal antiidiotype antibodies are prepared in accordance with the procedure of Examples 3 and 4, substituting anti-(endometriosis) antibody for the endometriosis antigen as the immunizing agent. The initial screening criterion is binding to anti-(endometriosis) antibody.

## EXAMPLE 6

### $^{125}$I-Labeled MS2B6 IgM Antibody

$^{125}$I-labeling was achieved using the Bolton-Hunter reagent described in A. Bolton et al, *Biochem.J.* **133**:529-1083 (1974). 250 $\mu$Ci of the reagent (Amersham) was evaporated, and the HPIEC-purified product of Example 1, dialyzed against 0.1 M sodium borate buffer, pH 8.5, was added. After 20 min at 0° C, 0.5 mL of 0.2 M glycine in borate buffer was added to stop the reaction. Unincorporated $^{125}$I was separated from $^{125}$I-labeled MS2B6 IgM by exclusion chromatography on Sephadex G-25 (Pharmacia). Specific activities in the range of 1 to 5 $\mu$Ci per Ig were obtained.

Substituting the antigen and antibodies of Examples 2, 3, 4 and 5 yields labeled endometriosis antigen, anti-(endometriosis) polyclonal and monoclonal antibodies, and antiidiotype antibodies, respectively.

## EXAMPLE 7

### Radioiodine Labeled MS2B6 IgM Antibody

Repeating the procedure of Example 2 but replacing the $^{125}$I reagent with the corresponding $^{123}$I and $^{131}$I reagents yields the corresponding $^{123}$I-labeled MS2B6 IgM and $^{131}$I-labeled MS2B6 IgM.

Substituting the antigen and antibodies of Examples 2, 3, 4 and 5 yields labeled endometriosis antigen, anti-(endometriosis) polyclonal and monoclonal antibodies, and antiidiotype antibodies, respectively.

14

## EXAMPLE 8

### Enzyme Labeled MS2B6 IgM Antibody

Horseradish peroxidase is conjugated to anti-(endometriosis) antibody of Example 1 in accordance with the procedure of H. Nygren et al, *Med.Biol.* **57**:187-191 (1979) as follows. Horseradish peroxidase (HRP, Type II or Type VI, Sigma) is dissolved in 0.05 M carbonate: bicarbonate buffer, pH 9.5, containing 0.25% glutaraldehyde (GA, Polaron). After 2 hr at room temperature, the excess GA is separated from the GA-HRP on a Sephadex G-25 column (0.7 12 cm, Pharmacia) equilibrated with 0.15 M NaCl. The GA-HRP complex is, in a second step, mixed with the antibody in 0.05 M carbonate: bicarbonate buffer, pH 9.5, containing 0.15 M NaCl, at different HRP ratios for 16-64 hr at 4°C. The reaction is stopped by the addition of lysine to a final concentration of 0.02 M.

Substituting the antigen and antibodies of Examples 2, 3, 4 and 5 yields labeled endometriosis antigen, anti-(endometriosis) polyclonal and monoclonal antibodies, and antiidiotype antibodies, respectively.

## EXAMPLE 9

### Radio-metal Labeled MS2B6 IgM Antibody

In this procedure the antibodies of Example 1 are conjugated with a chelating agent such as diethylenetriaminepentaacetic acid which is capable of forming a chelate with the metallic radionuclide. A suspension of 0.1 mg/mL of the bicyclic anhydride of DTPA (diethylenetriaminepentaacetic acid) is prepared in a dry solvent such as chloroform, ether or dry DMSO. An aliquot is removed to a clean, dry tube sufficient to provide a DTPA to immunoglobulin molar ratio of 1:1 and evaporated under nitrogen. A 10-20 microliter portion of the antibody solution of Example 1 (10-20 mg/mL) in 0.05 M bicarbonate buffer in saline, pH 7.0-7.5 is added to the dry DTPA, and the contents are agitated for 0.5-1.0 min. The coupled protein preparation is diluted to 0.2 mL with the same buffer solution and purified on a 5 cm gel filtration column with SEPHADEX G-50 gel, using a saline eluant. The coupling efficiency is determined before purification by the addition of "chelation-grade" $^{111}$In in 0.5 M acetate buffer solution, pH 6.0. Thin layer chromatography is used to separate the DTPA coupled antibody for calculation of the coupling efficiency. The DTPA-coupled antibodies are stored at 4°C until needed for binding with metallic radionuclides such as $^{111}$In$^{+3}$, $^{212}$Bi$^{+3}$, $^{99m}$Tc, and $^{68}$Ga$^{+3}$ to form the corresponding chelates.

Substituting the antigen and antibodies of Examples 2, 3, 4 and 5 yields labeled endometriosis antigen, anti-(endometriosis) polyclonal and monoclonal antibodies, and antiidiotype antibodies, respectively.

## EXAMPLE 10

### Biotin Labeled MS2B6 IgM Antibody

Biotin labeling was effected by the method of Haspel, M. et al, *Canc.Res.* **45**:3951-3961 (1985) with modifications. The HPIEC-purified produce of Example 1 at 1 mg/mL was dialyzed against 0.01 M potassium phosphate and 0.15 M potassium chloride, pH 7.8. Biotin: N-hydroxysuccinimide (Sigma) dissolved at 1 mg/mL in DMSO was added to give a molar ratio of biotin to IgM of 30:1 (using the molecular weight of monomer IgM of 180,000). After 15 min at room temperature with agitation, one-tenth volume of 1 M ammonium chloride was added, and the solution was dialyzed against PBS with 0.02 % sodium azide.

Substituting the antigen and antibodies of Examples 2, 3, 4 and 5 yields labeled endometriosis antigen, anti-(endometriosis) polyclonal and monoclonal antibodies, and antiidiotype antibodies, respectively.

## EXAMPLE 11

### Enzyme Labeled Anti-(endometriosis) Antibody

Anti-(endometriosis) antibody prepared in accordance with the procedures of Example 1 is conjugated with alkaline phosphatase following the one-step glutaraldehyde procedure Avrameas, *Immunochem.* 6:43 (1969), or following the modified procedure of M. O'Sullivan et al, *Anal.Biochem.*100:100 (1979), the entire contents of which are hereby incorporated by reference.

Substituting the antigen and antibodies of Examples 2, 3, 4 and 5 yields labeled endometriosis antigen, anti-(endometriosis) polyclonal and monoclonal antibodies, and antiidiotype antibodies, respectively.

## EXAMPLE 12

### Antibody Coated Microtiter Plate

Antibody prepared as described in Example 1 is diluted to 10 $\mu$g/mL in 0.05 M carbonate buffer, pH 9.6. 100 $\mu$L is dispersed into each well of of an IMMULON II microtiter plate (Dynatech). The plate is covered and incubated for 4 hr at room temperature or at 4° C overnight. The plate is washed 4 times with Wash Buffer (0.02 M Tris HCl, 0.015 M NaCl, 0.05% TWEEN-20), filling and emptying the wells completely with each use. The plate is then blocked by dispersing into each well 200 $\mu$L of a blocking solution (0.01 M PBS, 1% BSA, 0.02% NaN$_3$, pH 7.4) and incubating for 1 hr at room temperature. The wells are then washed 4 times with Wash Buffer, as described above. The plate is now ready for immunoassay of samples.

Substituting the antigen and antibodies of Examples 2, 3, 4 and 5 yields microtiter plates including endometriosis antigen, anti-(endometriosis) polyclonal and monoclonal antibodies, and antiidiotype antibodies, respectively.

## EXAMPLE 13

### Sandwich Immunoassay

Positive and negative controls are included in the test. The negative control is a serum sample of a patient known to be free of endometriosis. A serum sample from a male human can be used. The positive control can be a sample including the antigen of Example 2. A standard using endometriosis antigen serially diluted in sample diluent provides a standard curve.

A microtiter plate prepared as in Example 12 with the MS2B6 antibody is used for the assay. 100 $\mu$L of each standard, sample, positive and negative control are placed in separate wells and incubated 2 hr at room temperature. The plate is washed 4 times with Wash Buffer as described in Example 11. 100$\mu$L of alkaline phosphatase-conjugated goat anti-(endometriosis) antibody prepared as in Example 11 is diluted 1/1000 in Conjugate Buffer (0.02 M Tris-HCl, pH 8, 0.03 M NaCl, 0.05% TWEEN 20, 5% BSA, 0.02% NaN$_3$). 100 $\mu$L is dispersed into each well and incubated for 2 hr at room temperature. The plate is washed 4 times as previously described. 4 mg/mL of p-nitrophenylphosphate (PNPP) is used as the substrate. This is diluted in 0.18 M 2-amino-2-methyl-1-propanol (AMP) buffer, pH 9.5 with 0.12 mM mgCl$_2$. 100 $\mu$L is dispersed into each well of the microtiter plate. After a 5 min incubation at room temperature, the reaction rate in milli-OD/min is read at 405 nm on a V-MAX™ microtiter plate reader (Molecular Devices).

A standard curve is constructed by correlating increasing reaction rate with increasing endometriosis antigen concentration in the standards. Unknowns are calculated directly from the curve or by using a pre-set computer program (Molecular Devices).

To independently test for MS2B6 IgM binding to elements of the blood (e.g., RBC, lymphocytes, granulocytes, platelets), further testing was performed. MS2B6 IgM at 50 $\mu$g/mL was tested by the Stanford Transfusion Service using standard hemagglutination techniques. MS2B6 IgM failed to react with a panel of red blood cells expressing the following antigens: Blood group antigens A and B; Rh antigens D, C, c, E and e; Kell antigens K, k, $K^b_p$ and $J^b_s$ ; Duffy antigens $F^a_y$ and $F^b_y$; Kidd antigens $J^a_k$ and $J^b_k$; Lewis antigens $Le^a$ and $Le^b$; MN antigens M, N, S and s; and Lutheran antigens $Lu^a$, and $Lu^b$; and $X^a_g$ antigen.

## EXAMPLE 14

### Immunoperoxidase Studies

Tissues are obtained after surgery or autopsy. The fresh tissues are cut into small pieces, wrapped in foil and stored in liquid nitrogen until immediately prior to transfer to embedding medium. Six micron cryostat sections are air dried and stored at 4°C, then fixed immediately prior to use with PLP fixative using the procedure of I. McLean et al, *J.Histochem.Cytochem.* **22**:1077-1083 (1974a) (0.5% paraformaldehyde, 0.075 M L-lysine, and 0.01 M sodium periodate). The sections are fixed for 10 min at 4°C followed by washing in PBS. Some tissues require blocking of endogenous biotin following the procedure of G. Wood et al, *J.Histochem.Cytochem.* **29**:1196-1204 (1981). Suppression of endogenous peroxidase activity is effected by the procedure of J. Kelley et al, *J.Immunol.Meth.* **96**:127-132 (1987). Immunoperoxidase staining with biotin-labeled MS2B6 IgM or control IgM is performed in the manner of W. Hancock et al, *Meth.Enzymol.* **121**:828-848 (1986), briefly as follows. The sections are sequentially incubated (with PBS washing between) with 10% fetal calf serum to block, biotin-labeled antibody at 30 $\mu$g/mL, streptavidin-HRP (Sigma) at 0.5 $\mu$g/mL, then diaminobenzidine (Sigma) at 1 mg/mL with 0.03% hydrogen peroxide. After 15 min, slides are washed with water, counterstained with hematoxalin and coverslipped. Microscopically, positive staining by biotin-labeled MS2B6 IgM is visualized as deposition of brown stain on cells not stained by biotin-labeled control IgM.

Fixation of the sections with paraformaldehydelysine-periodate allows adequate tissue preservation as well as antigen staining. Gluteraldehyde (0.1%) and neutral formalin (2%) preserve some antigen staining, but methanol fixation is not effective, destroying antigen staining.

## EXAMPLE 15

### Enzyme Immunoassay Studies

The procedure of Example 13 is repeated using, as a positive control, the antigen of Example 2. A standard using endometriosis antigen serially diluted in sample diluent provides a standard curve. A microtiter plate prepared as in Example 12 with the antibody of Example 4 is used for the assay.

A standard curve is constructed by correlating increasing reaction rate with increasing endometriosis antigen concentration in the standards. Unknowns are calculated directly from the curve or by using a pre-set computer program (Molecular Devices).

## EXAMPLE 16

### EA 101 Endometriosis Antigen Preparation

EA 101 Endometriosis antigen is a combination of EA 101A, EA 101B and EA 101C. EA 101A and EA 101B are the fractions obtained from the culture medium extracts referred to as EA 101D.

The method of detecting endometriosis by this method involves the determination of the levels of antibodies against certain antigens (EA 101A and EA 101B) which were isolated from culture medium in which epithelial cells were grown in vitro using tissue culture medium consisting of 1 - 10 % Fetal bovine serum or serum free medium or monolayer cultures of epithelial cells (EA 101C). The epithelial cell line was derived from ovarian carcinoma and similar antigens are also present in breast, colon and pancreatic cancer. All the experimental results in the study were carried out using an ovarian epithelial carcinoma cell line No. 2774 since this cell line appears to produce more antigens compared to other cell lines.

Isolation of EA 101D:

The epithelial cell lines were grown in MEM alpha medium containing 1-10% fetal bovine serum for 3 to 7 days and the culture medium was collected and centrifuged for 10 minutes at 2000 rpm in an IEC centrifuge and the clear supernatant was processed in any one of the following pprocedures.

i) The medium was dialyzed against Phosphate buffered saline and then concentrated by ultrafiltration using Amicon YM 10 membrane. The concentrated solution was then fractionated by Affinity chromatography.

ii) The medium was precipitated by 50% saturated ammonium sulfate solution and centrifuged at 3000 rpm. The precipitate was dissolved in PBS and dialized against PBS. The dialyzed preparation was fractionated by Affinity chromatography.

iii) The medium was precipitated with cold actone at $20^{\circ}$ C and centrifuged. The precipitate was dried, dissolved in PBS, and fractionated by Affinity chromatography.

iv) The medium was dialyzed against deionized water, lyophilized and dissolved in a small volume of water, precipitated with cold acetone at -20. C and centrifuged. The precipitate was dissolved in water and lyophylized. The lyophylized powder was fractionated by Affinity chromatography.

Affinity chromatographic isolation of 101A and 101B:

The samples obtained from the medium were applied to Affigel Blue column, equilibrated with PBS, and the unbound proteins (Flow through) were collected and concentrated by Amicon YM 10 ultrafiltration. This fraction is referred to as EA 101B. The bound proteins were eluted by 1.5 M sodium chloride solution in PBS, dialyzed against PBS, and then concentrated by Amicon YM 10 ultrafiltration. The concentrated proteins were applied to an affinity column to remove albumin. The column was prepared by coupling goat-anti-bovine serum albumin antibody (affinity purified) to cyanogen bromide activated sepharose 4B. The antigens obtained after removal of Albumin are referred to as EA 101A.

Isolation of 101C:

The cytoplasmic proteins from monolayer cultures of epithelial cells are extracted as described by Fey, et al, *J.Cell Biol.* 98:1973 (1984). The proteins were isolated by PIPES buffer containing 0.1% Triton-X-100 at $4^{\circ}$ C for 10 minutes. The antigens were isolated from the extracts as follows. The extract is first dialyzed against PBS and then remaining Triton-X-100 is removed by passing through SM-2 Biobeads. The antigen preparation after removal of Triton-X-100 is then purified further by gel filtration to remove proteins with molecular weights less than 50 KD. The higher molecular weight proteins, referred to as EA 101C, are used as antigens to detect the antibodies in sera of patients with endometriosis.

**EXAMPLE 17**

*EA 101 Endometriosis Antigen*

18

The active antigens which react with the antibodies present in the sera of endometriosis patients are characterized by separating the proteins from EA 101A, EA 101B and EA 101C by 7.5% SDS-Polyacrylamide gel electrophoresis and Western Blotting. The EA 101 preparations were applied to the SDS-PAGE gels at concentrations from 5 to 20 µg and electophoresed in a Mighty small gel electrophoresis unit (Hoefer Scintific Co.) using Laemmli's method, *J.Mol. Biol.* **80**:575 (1973). The proteins were transferred to a nitrocellulose membrane by the procedure of Towbin, et al, *Proc.Natl.Acad.Sci.(U.S.A.)* **76**:4350 (1979). The Blots were saturated with 1% BSA, 1% casein and 1% non-fat milk powder in PBS and then reacted with Patients' sera, 20-40 times diluted with PBS-BSA (1%)-Tween 20 (0.5%). Sera were from patients with endometriosis confirmed by laparoscopy or from apparently healthy patients. The specific antigens were then identified by reacting with biotinylated anti-human IgG (diluted 500 times with PBS-Tween 20 (0.05%), followed by Streptavidin horse radish peroxidase and then with 4-chloro-1-naphthol (0.05% in Tris buffered saline, pH 7.6) and hydrogen peroxide (0.015%). The molecular weights of the antigens, which are visualized by the colored bands on the blots, were determined from a standard curve constructed by plotting the Log of molecular weight standards against relative mobilities ($R_f$).

Based on the results of these experiments, the following antigens are the most important and significant for the detection of antibodies in sera of endometriosis patients, since these antigens reacted with endometriosis patients' sera and not with control sera.

EA 101 A: 73 KD
EA 101B: 50.2 KD and 57.7 KD
EA 101C: 65.7 KD and 145.3 KD
EA 101 D: 96.8 KD, 152 KD and 173 KD.

## EXAMPLE 18

### Endometriosis Immunoassay

### Microtiter Plate Preparations

Antigens EA 101A, EA 101B and EA 101C are coated on the 96 well microtiter plates either separately or in different combinations at concentrations ranging from 0.1 - 10 ug/ml of coating buffer by incubating with antigen solution at 4°C for 16 hours or at room temperature for 4 hours. The plates are washed twice with Tris-buffered saline (TBS) and then incubated with 1% BSA in TBS for two hours at 37°C to block remaining binding sites. The plates are washed with TBS-Tween 20 (0.05%) four times.

## EXAMPLE 19

### ELISA Endometriosis Assay

Patients' sera were diluted 50 to 800 times with serum diluent (PBS-1% BSA-0.5% Tween 20-300 mM sodium chloride), and 100 - 200 µl/well was added to the microtiter plate described in Example 18. The plate was incubated at 37°C for 2 hours. (The incubation time can be varied from 15 min to 3 hours and the temperature can be room temperature). After the incubation, the plate was washed with Tris buffered saline containing 0.05% Tween 20, and incubated with 100 - 200 µl of alkaline phosphatase conjugated goat-anti-human IgG as the conjugate. The activity was determined by reacting with *p*-nitrophenyl phosphate as the substrate.

The levels of specific antibodies in endometriosis patients' sera are determined by subtracting the values obtained when no antigen was coated on the plate.

## EXAMPLE 20

*Endometriosis Antigen Diffraction*

*Biograting Preparation*

A polysilicon coated silicon wafer is immersed for 2 hrs or overnight in a solution of an endometriosis antigen (EA 101A antigen, EA 101B antigen, EA 101C antigen or 1:1:1 molar ratios thereof), diluted in 0.1 M phosphate buffered solution, pH 7.5, with 0.1 wt.% sodium azide preservative. The wafer is then removed from the antigen solution, rinsed with buffered saline, and dried.

A mask having a series of diffraction grating lines having a line width, w, of 25 $\mu$m, and a line spacing, d, of 25 $\mu$m is placed in a UV exposure device (Karl Suss Mask Aligner), and exposed to ultraviolet light having a wavelength of 254 nm and a strength of 9.6 milliwatts per $cm^2$ for 3 min.

The wafer is then immersed in a 0.1 M phosphate buffer solution (PBS), pH 7.4, containing 2.5 wt.% sucrose, 0.25 wt.% bovine serum albumin (BSA) and 0.1 wt.% sodium azide for 30 min, the excess is removed, and the wafer is dried.

The wafer is then cut into square chips having a diffraction grating pattern of endometriosis antigen thereon, and mounted on the surface of a polyethylene dipstick.

## EXAMPLE 21

*Endometriosis Biograting Immunoassay*

Patient serum is added to the surface of a polysilicon chip product of Example 20 and incubated for one hr. The serum is removed, and the chip is rinsed thoroughly with distilled water, and excess water is removed. The diffraction grating pattern of active endometriosis antigen binds with endometriosis antibody in the serum.

The intensity of the light diffracted by the grating is then measured using monochromatic light having a wavelength of 632.8 nm from a helium-neon laser.

The procedure is repeated with a positive control containing a known amount of endometriosis antibody to establish a reference point cut off with which the intensity of the diffracted light obtained with the patient serum sample is compared to determine the presence or absence of endometriosis.

## EXAMPLE 22

*Endometriosis ELISA Immunoassay*

An ELISA microtiter plate was coated with endometriosis antigen EA 101A by the procedure of Example 18, using a concentration of 3 $\mu$g/ml endometriosis antigen. An ELISA immunoassay was carried out following the procedure of Example 19 with serum from patients who had been laparoscoped to confirm the presence or absence of endometriosis. The patient serum was diluted 100 times with serum diluent, and the results were determined with a V-MAX™ microtiter plate reader (Molecular Devices). The levels of antibodies in the samples were obtained in units of mOD/min. Values below 7 mOD/min were considered negative, and values above 7 were considered positive. The positive values varied between 7 and 150 mOD/min. The results are shown in Table C.

TABLE C

| ELISA ENDOMETRIOSIS TEST RESULTS | | |
|---|---|---|
| Patients | Endometriosis | Control |
| Total No. | 62 | 134 |
| True Positive | 39 | --- |
| True Negative | --- | 106 |
| False Positive | --- | 28 |
| False Negative | 23 | --- |
| Sensitivity : 63% | | |
| Specificity: 79% | | |

**EXAMPLE 23**

*Endometriosis ELISA Immunoassay*

The procedure of Example 19 was repeated with sera from normal patients and patients with known gynecologic disorders other than endometriosis. The results are shown in Table D.

TABLE D

| Patients | Total No. | True Negative | False Positive |
|---|---|---|---|
| PID | 40 | 35 | 5 |
| Cancer (GYN) | 7 | 6 | 1 |
| Normal | 41 | 36 | 5 |
| Infertility | 25 | 21 | 4 |
| GYN Disorders | 19 | 13 | 6 |
| SLE | 2 | 2 | 0 |
| Pregnancy | 13 | 12 | 1 |
| Pre-eclampsia | 10 | 8 | 2 |
| PID - Pelvic inflammatory Disease | | | |
| SLE - Systemic lupus Eryrthromatosis | | | |

**Claims**

1. A method for determining the presence of endometriosis in a patient comprising
   (a) contacting a sample from the patient with an anti-(endometriosis) antibody, and
   (b) determining binding of the antibody with a component of the sample.
2. A method according to claim 1 wherein the anti-(endometriosis) antibody is a human monoclonal antibody.
3. A method according to claim 3 wherein the ant-(endometriosis)antibody is MS2B6 IgM antibody.
4. A method for determining the presence of endometriosis in a patient comprising
   (a) contacting a blood serum sample from the patient with endometriosis antigen or endometriosis antiidiotype antibody and

(b) determining binding of endometriosis antigen or endometriosis antiidiotype antibody with antibody in the sample.

5. An immunoassay kit for detecting endometriosis comprising an anti-(endometriosis) antibody.

6. The immunoassay kit of claim 5 wherein the anti-(endometriosis) antibody is a human monoclonal antibody.

7. The immunoassay kit of claim 5 wherein the anti-(endometriosis) antibody is labeled MS2B6 IgM antibody.

8. An antibody which binds preferentially with endometriosis antigen.

9. An antibody of claim 8 which is a human monoclonal antibody.

10. An antibody of claim 9 which is MS2B6 IgM antibody.

11. An endometriosis antigen consisting of EA 101A antigen, EA 101B antigen, EA 101C antigen, or mixtures thereof.

12. An endometriosis antigen reagent consisting essentially of an endometriosis antigen of claim 11 bound to an insoluble support.

13. An endometriosis antigen reagent of claim 12 wherein the insoluble support is a microwell.

14. A biograting comprising alternating active and deactivated zones of antigen of claim 11.

15. A biograting comprising alternating active and deactivated zones of antibody of claim 8.